# EUROPÄISCHE PATENTSCHRIFT

(11) **EP 0 883 378 B1**
(45) Veröffentlichungstag und Bekanntmachung des Hinweises auf die Patenterteilung: **16.05.2001**
(21) Anmeldenummer: 97902347.0
(22) Anmeldetag: 07.02.1997
(51) Int. Cl.: A61B 17/16, B23B 45/02, B25F 5/02

(54) **BOHRMASCHINE FÜR CHIRURGISCHE ZWECKE**
DRILL FOR SURGICAL USE
PERCEUSE A USAGE CHIRURGICAL

(30) Priorität: 26.02.1996 DE 19607123
(43) Veröffentlichungstag der Anmeldung: 16.12.1998
(73) Patentinhaber: Aesculap AG & Co. KG, 78532 Tuttlingen (DE)
(72) Erfinder: HÖGERLE, Roland, Alois, D-78532 Tuttlingen (DE)
(74) Vertreter: Böhme, Ulrich, Dr. Dipl.-Phys.
(86) Internationale Anmeldenummer: EP9700549
(87) Internationale Veröffentlichungsnummer: WO9731576

(56) Entgegenhaltungen:
- EP-A- 0 275 392
- US-A- 4 091 880

## Beschreibung

Die Erfindung betrifft eine Bohrmaschine für chirurgische Zwecke mit einem Gehäuse, in dem ein Elektromotor und ein von diesem angetriebener Bohrmechanismus angeordnet sind, und mit einem ein Teil des Gehäuses bildenden Handgriff, an dem Steuergriffe zur Geschwindigkeitssteuerung und zur Richtungsumschaltung des Elektromotors angeordnet sind und der zur Aufnahme einer Batterie und der Steuerelektrik des Elektromotors dient.

Bei chirurgischen Handbohrmaschinen ist es notwendig, diese Geräte für jeden Einsatz zu sterilisieren. Die Sterilisierung erfolgt üblicherweise durch eine Dampfbehandlung bei erhöhter Temperatur, und dieser Sterilisiervorgang kann für einzelne Bestandteile einer derartigen Bohrmaschine schädlich sein, insbesondere gilt dies für elektrische Teile.

Es ist eine chirurgische Handbohrmaschine bekannt, bei der in einem abnehmbaren Handgriff eine komplette Antriebseinheit angeordnet ist, die aus Batterie, Steuerelektrik und Elektromotor besteht und die zusammen mit dem Handgriff von dem Gehäuse abnehmbar ist (DE 33 17 398 A1). Bei dieser vorbekannten Handbohrmaschine muß in komplizierter Weise eine mechanische Kupplung zwischen dem Elektromotor im Handgriff einerseits und dem Antriebsmechanismus im Gehäuse der Bohrmaschine andererseits vorgenommen werden. Nachteilig ist bei der bekannten Handbohrmaschine auch, daß die Abmessung des Elektromotors beschränkt ist, da im Handgriff aus ergonomischen Gründen wenig Platz für die Aufnahme des Elektromotors gegeben ist. Daher können auf diese Weise nur relativ schwache Handbohrmaschinen aufgebaut sein, die beispielsweise zum Eindrehen eines Bohrdrahts eingesetzt werden können.

Die EP-A-275 392 zeigt eine Bohrmaschine, wie der Motor in dem Gehäuse eingebaut ist und dadurch platz in dem Griff schafft.

Ausgehend von diesem Stand der Technik ist es Aufgabe der Erfindung, eine Bohrmaschine der gattungsgemäßen Art so auszubilden, daß einerseits kräftige Antriebsmotoren verwendet werden können und andererseits sichergestellt werden kann, daß die empfindlichen Teile der Handbohrmaschine durch den Sterilisiervorgang nicht beschädigt werden.

Diese Aufgabe wird bei einer Bohrmaschine der eingangs beschriebenen Art erfindungsgemäß dadurch gelöst, daß in den Handgriff ein Energieblock einsetzbar ist, der die Batterie, die Steuerelektrik, von den Steuergriffen betätigbare Schalter für die Geschwindigkeitssteuerung und Richtungsumschaltung und elektrische Anschlüsse für den im Gehäuse angeordneten Elektromotor umfaßt.

Bei dieser Bohrmaschine wird also ein Energieblock verwendet, der im Prinzip weitgehend aufgebaut ist wie eine herkömmliche Batterie und der auch in ähnlicher Weise in den Handgriff eingesetzt werden kann. Dieser Energieblock umfaßt aber neben der eigentlichen Batterie auch noch die Steuerelektrik sowie Schalter für die Geschwindigkeitssteuerung und die Richtungsumschaltung, die ihrerseits von den Steuergriffen am Handgriff betätigbar sind. Dieser Energieblock wird mit der übrigen

Bohrmaschine ausschließlich durch elektrische Anschlüsse verbunden, also in ähnlicher Weise wie eine einsetzbare Batterie, jedoch wird über die elektrischen Anschlüsse ein Steuerstrom für den Elektromotor abgegeben, der durch die Steuerelektrik entsprechend der jeweiligen Stellung der Schalter für Geschwindigkeitssteuerung und Richtungsumschaltung geformt ist. Der Energieblock gibt also je nach Betriebsbedingungen unterschiedliche Ströme ab und bewirkt auf diese Weise, daß der Elektromotor, der im Gehäuse selbst verbleibt, wahlweise in der einen oder in der anderen Richtung umläuft und mit der jeweils gewünschten Geschwindigkeit.

Bei dieser Konstruktion kann der Energieblock als vollständig abgeschlossenes Gehäuse aufgebaut sein, wie man dies an sich von Batterien her kennt, alle übrigen Teile verbleiben an der Bohrmaschine und können mit dieser sterilisiert werden, dies gilt insbesondere für den gesamten Handgriff einschließlich der die Schalter betätigenden Steuergriffe.

Beim Zusammensetzen der Bohrmaschine wird dieser Energieblock über einen sogenannten Steriltrichter in den Handgriff eingesetzt und dieser anschließend verschlossen, so daß die Handbohrmaschine nach außen hin vollständig steril ist, lediglich der Innenraum des Handgriffs bildet einen unsterilen Bereich. Dies ist jedoch in keiner Weise störend, da der Innenraum des Handgriffs vollständig abschließbar ist, insbesondere wird zur übrigen Handbohrmaschine kein Durchbruch benötigt, da in diesem Bereich lediglich elektrische Verbindungen vorzusehen sind.

Besonders günstig ist es, wenn der Energieblock eine untere Kammer für die Batterie und eine obere Kammer für die Steuerelektrik aufweist. Es ergeben sich auf diese Weise besonders kurze Verbindungswege für die elektrischen Leitungen, da an der Oberseite des Energieblocks die elektrische Verbindung zum Gehäuse und damit zum Elektromotor angeordnet werden kann.

Günstig ist es weiterhin, wenn die Schalter für die Geschwindigkeitssteuerung und die Richtungsumschaltung in der oberen Kammer angeordnet sind, das heißt die Schalter werden dann unmittelbar mit der Steuerelektrik kombiniert.

Die Einsetzbarkeit des Energieblocks wird besonders einfach, wenn der Energieblock durch die geöffnete Unterseite des Handgriffs in diesen einschiebbar ist.

Bei einer bevorzugten Ausführungsform der Erfindung ist vorgesehen, daß der Energieblock die Schalter betätigende Betätigungsglieder aufweist, an denen bei eingeschobenem Energieblock die Steuergriffe anliegen und diese Betätigungsglieder durch ihre eigene Bewegung bewegen. Dadurch wird also eine mechanische Bewegung der Steuergriffe auf die Betätigungsglieder am Energieblock übertragen, die ihrerseits die Schalter betätigen, die im Inneren des Energieblocks angeordnet sind.

Vorzugsweise sind die Betätigungsglieder federbelastet in eine Ausgangsstellung bewegbar, gegen die Belastung dieser Federn können die Betätigungsglieder dann durch die Steuergriffe verschoben werden. Dies führt dazu, daß die federbelasteten Betätigungsglieder die Steuergriffe ihrerseits in die Ausgangsstellung zurücktreiben.

Bei einer bevorzugten Ausführungsform kann vorgesehen sein, daß ein Betätigungsglied an der Oberseite des Energieblocks aus diesem hervorsteht und quer zur Längsrichtung des Handgriffs verschiebbar am Energieblock gelagert ist.

Es kann weiterhin vorgesehen sein, daß ein Betätigungsglied im Inneren des Energieblocks quer zur Längsrichtung des Handgriffs verschiebbar gelagert ist und daß in der Vorderseite des Energieblocks eine Durchbrechung vorgesehen ist, durch die hindurch ein Steuergriff und das Betätigungsglied aneinander anliegen.

Diese Anlage kann gegebenenfalls abgedichtet sein, beispielsweise durch eine flexible Membran.

Besonders günstig ist es, wenn die Steuergriffe einen gegenseitigen Verriegelungsmechanismus aufweisen, der eine gleichzeitige Betätigung beider Steuergriffe verhindert. Dies stellt sicher, daß während des normalen Betriebs eine Richtungsumkehr unmöglich ist und daß außerdem der Motor nicht in Betrieb gesetzt werden kann, wenn die Richtungswahl noch nicht beendet ist.

Bei einer bevorzugten Ausführungsform ist vorgesehen, daß die Steuergriffe durch Öffnungen in der vorderen Wand des Handgriffs in dessen Innenraum eintauchen und daß die Öffnungen durch die Steuergriffe umgreifende flexible Membranen verschlossen sind. Insbesondere können die Membranen die Form eines Faltenbalgs aufweisen. Dadurch läßt sich der Innenraum des Handgriffs gegenüber dem Außenraum vollständig abschließen, obwohl bewegbare Teile durch Öffnungen in den Innenraum eintauchen. Dies ist zur Herstellung der Sterilität der Handbohrmaschine von wesentlicher Bedeutung.

Die nachfolgende Beschreibung bevorzugter Ausführungsformen der Erfindung dient im Zusammenhang mit der Zeichnung der näheren Erläuterung. Es zeigen:
- Figur 1:: eine schematische Seitenansicht einer Handbohrmaschine mit einem Handgriff und darin eingesetztem Energieblock;
- Figur 2:: eine Längsschnittansicht des Handgriffs mit eingesetztem Energieblock im Bereich der Steuergriffe mit dem Richtungsschalter in einer Stellung und dem Betriebsschalter in Arbeitsstellung;
- Figur 3:: eine Ansicht ähnlich Figur 2 mit dem Richtungsschalter während der Umschaltung und dem Betriebsschalter in Ruhestellung und
- Figur 4:: eine Ansicht ähnlich Figur 2 mit dem Richtungsschalter in der anderen Stellung und dem Betriebsschalter in Arbeitsstellung.

Die in der Zeichnung dargestellte Bohrmaschine 1 umfaßt ein Gehäuse 2, in dem in der Zeichnung nur schematisch dargestellt ein Elektromotor 3 und ein Antriebsmechanismus 4 für ein Bohrfutter 5 angeordnet sind. Diese Elemente können von beliebiger Bauart sein, wie sie dem Fachmann an sich bekannt sind.

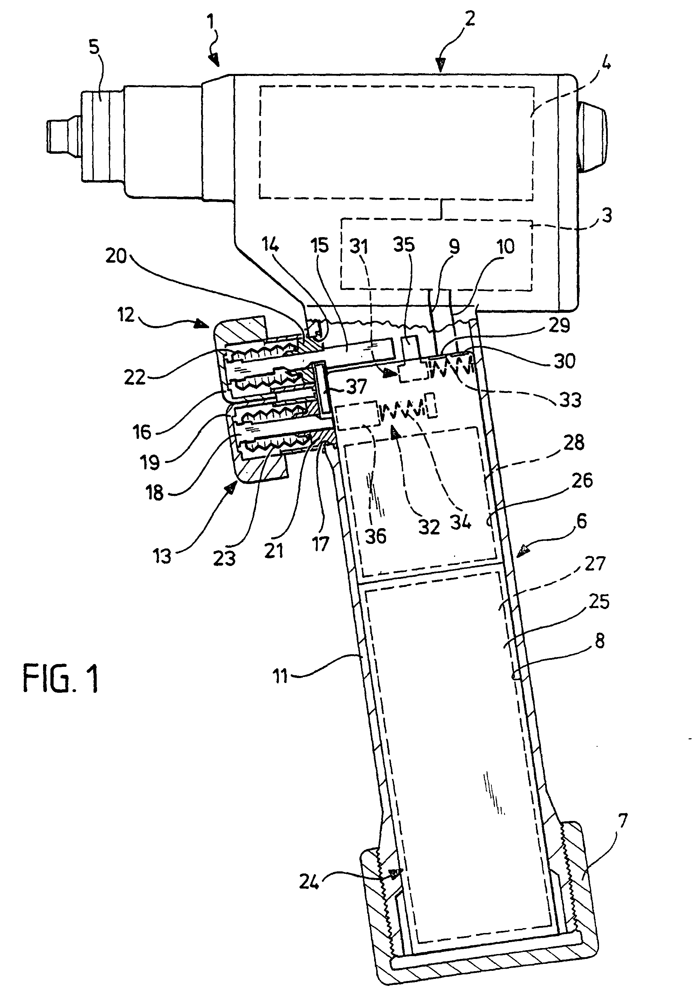

Am unteren Ende des Gehäuses 2 schließt sich an dieses ein quaderförmiger länglicher Handgriff 6 an, der an seiner Unterseite durch einen aufschraubbaren oder aufsteckbaren Deckel 7 verschlossen ist. Der Innenraum 8 des Handgriffs 6 ist gegenüber dem Innenraum des Gehäuses 2 durch eine in der Zeichnung nicht dargestellte Trennwand abgeschlossen, in diesem Bereich treten zwei in Figur 1 nur schematisch dargestellte elektrische Verbindungen 9, 10 aus dem Innenraum 8 des Handgriffs 6 in den Innenraum des Gehäuses 2 ein.

In der Vorderwand 11 des Handgriffs 6 befinden sich an dessen oberem Ende unmittelbar neben dem Gehäuse 2 übereinander zwei Steuergriffe, nämlich ein oberer Richtungstaster 12 und ein unterer Betriebstaster 13.

Der Richtungstaster 12 umfaßt einen durch eine Öffnung 14 durch die Vorderwand 11 in den Innenraum 8 eintre-tenden, in der Öffnung 14 längsverschieblich gelagerten Stößel 15, dessen freies Ende mit einer Griffhaube 16 verbunden ist, und in gleicher Weise weist der Betriebstaster 13 einen durch eine Öffnung 17 in den Innenraum 8 eintretenden, in dieser längsverschieblich gelagerten Stößel 18 auf, der seinerseits mit einer Griffhaube 19 verbunden ist. Beide Stößel 15 und 18 sind in Einsätzen 20 beziehungsweise 21 gelagert, die in die entsprechenden Öffnungen 14 beziehungsweise 17 eingesetzt sind. Beide Stößel 15 und 18 sind jeweils von einem zylindrischen Faltenbalg 22 beziehungsweise 23 umgeben, der beispielsweise aus einem gummielastischen Material bestehen kann und der einerseits an dem jeweiligen Stößel 15, 18 und andererseits an dem jeweiligen Einsatz 20 beziehungsweise 21 festgelegt ist, so daß damit die Öffnungen 14 und 17 dicht verschlossen sind. Trotzdem lassen sich die Stößel 15 und 18 in den Öffnungen 14 beziehungsweise 17 verschieben.

In den Innenraum 8 des Handgriffs 6 ist ein quaderförmiger Energieblock 24 eingeschoben, dessen Innenraum in eine untere Kammer 25 und in eine obere Kammer 26 unterteilt ist. In der unteren Kammer 25 ist eine herkömmliche, vorzugsweise wiederaufladbare Batterie 27 angeordnet, in der oberen Kammer 26 befindet sich eine Steuerelektronik 28, wobei sowohl die Batterie 27 als auch die Steuerelektronik 28 in der Zeichnung nur schematisch und gestrichelt wiedergegeben sind.

Die Steuerelektrik 28 steht einerseits elektrisch mit der Batterie 27 in Verbindung und andererseits über die Leitungen 9 und 10 mit dem Elektromotor 3. Dazu sind an der Oberseite des Energieblocks 24 zur Herstellung einer elektrischen Verbindung mit den Leitungen 9 und 10 elektrische Kontakte 29 und 30 vorgesehen, die beim Einschieben des Energieblocks 24 in den Innenraum 8 automatisch die elektrische Verbindung mit den Leitungen 9 und 10 ausbilden.

In der oberen Kammer 26 befinden sich außerdem ein Richtungsumschalter 31 sowie ein Schalter 32, der die Stromzufuhr zum Elektromotor 3 ein- und ausschaltet und der außerdem Schaltelemente in der Steuerelektronik 28 so verstellt, daß die an den Elektromotor 3 abgegebene Betriebsspannung variiert wird und somit den Elektromotor 3 mit unterschiedlicher Drehzahl antreibt. Derartige Schaltelemente sind an sich bekannt und werden daher nicht näher erörtert.

Der Richtungsumschalter 31 umfaßt ein Betätigungsglied 35, welches im Inneren der oberen Kammer 26 gegen die Wirkung einer Feder 33 verschiebbar gelagert ist und nach oben über den Energieblock 24 hervorsteht. In diesem Bereich liegt der Stößel 15 an dem Betätigungsglied 35 an, so daß das Betätigungsglied 35 gegen die Wirkung der Feder 33 verschoben wird, wenn der Stößel 15 in den Innenraum 8 eingeschoben wird. Dabei betätigt das Betätigungsglied 35 einen in der Zeichnung nicht näher dargestellten Schalter, der die Richtung des Elektromotors umkehrt, der also in einer Endstellung die Steuerelektronik 28 für den Betrieb des Elektromotors in einer Richtung zubereitet, in der anderen Endstellung für die entgegengesetzte Richtung.

Ebenfalls in der oberen Kammer 26 ist ein weiteres Betätigungsglied 36 in der Verlängerungsrichtung des Stößels 18 verschiebbar gelagert, welches seinerseits über eine Feder 34 gegen den Stößel 18 geschoben wird, so daß dieser unter der Wirkung der Feder 34 in die ausgeschobene Stellung verschoben wird. Durch Einschieben des Stößels 18 kann das Betätigungsglied 36 entgegen der Wirkung der Feder 34 verschoben werden. Bei entspannter Feder 34 ist der Elektromotor ausgeschaltet, beim Einschieben entgegen der Wirkung der Feder 34 wird die Steuerelektronik 28 so verändert, daß bei zunehmender Einschubtiefe des Stößels 18 die Drehzahl des Elektromotors 3 erhöht wird.

Da die Stößel 15 und 18 nur an den entsprechenden Betätigungsgliedern 35 beziehungsweise 36 anliegen, kann der Energieblock 24 jederzeit nach unten aus dem Handgriff 6 herausgenommen und nach dem Laden der Batterie wieder eingesetzt werden, nach dem Einschieben des Energieblocks 24 ist die Bohrmaschine sofort wieder betriebsbereit.

Um zu verhindern, daß während des Betriebs des Elektromotors die Richtung umgeschaltet wird und daß der Elektromotor in Betrieb genommen werden kann, wenn die Richtungsumschaltung noch nicht beendet worden ist, ist ein spezieller Verriegelungsmechanismus vorgesehen. Dieser umfaßt einen quer zur Verschieberichtung der Stößel 15 und 18 zwischen diesen verschieblichen gelagerten Stift 37, dessen Länge geringfügig größer ist als der Abstand zwischen den beiden Stößeln 15 und 18. Der obere Stößel 15 weist zwei im Abstand zueinander angeordnete, dem anderen Stößel 18 zugewandte Ausnehmungen 38 und 39 auf, der Stößel 18 seinerseits eine dem Stößel 15 zugewandte einzige Ausnehmung 40.

Alle Ausnehmungen 38, 39 und 40 sind einseitig mit einer Aufgleitfläche 41, 42 beziehungsweise 43 versehen, durch die der in die entsprechende Ausnehmung eintauchende Stift 37 beim Verschieben des entsprechenden Stößels aus der entsprechenden Ausnehmung ausgehoben werden kann. Dieses Ausheben ist aber nur dann möglich, wenn in dem jeweils anderen Stößel eine Ausnehmung gegenüberliegt, ist dies nicht der Fall, stößt der Stift an dem entsprechenden Stößel an und verhindert ein Ausheben des Stifts aus der Ausnehmung und damit auch eine Verschiebung des Stößels, in dessen Ausnehmung der Stift eintaucht.

Bei der Darstellung der Figur 1 befinden sich beide Stößel 15 und 18 unter dem Einfluß der Federn 33 und 34 in der ausgeschobenen Stellung, und in dieser Stellung ist die Ausnehmung 40 des unteren Stößels 18 ebenso mit dem Stift 37 ausgerichtet wie die dem Handgriff 6 näherliegende Ausnehmung 39 des Stößels 15. Ausgehend von dieser Ruhestellung kann der Benutzer wahlweise den Richtungstaster 12 oder den Betriebstaster 13 betätigen, der Stift 37 wird dabei in die Ausnehmung des jeweils anderen, dann unbetätigten Stößels eingeschoben werden.

Werden beide Stößel gleichzeitig betätigt ist dies nicht möglich, da dann der Stift 37 nicht in eine der Ausnehmungen eintauchen kann, gleichzeitige Betätigung wird also blockiert.

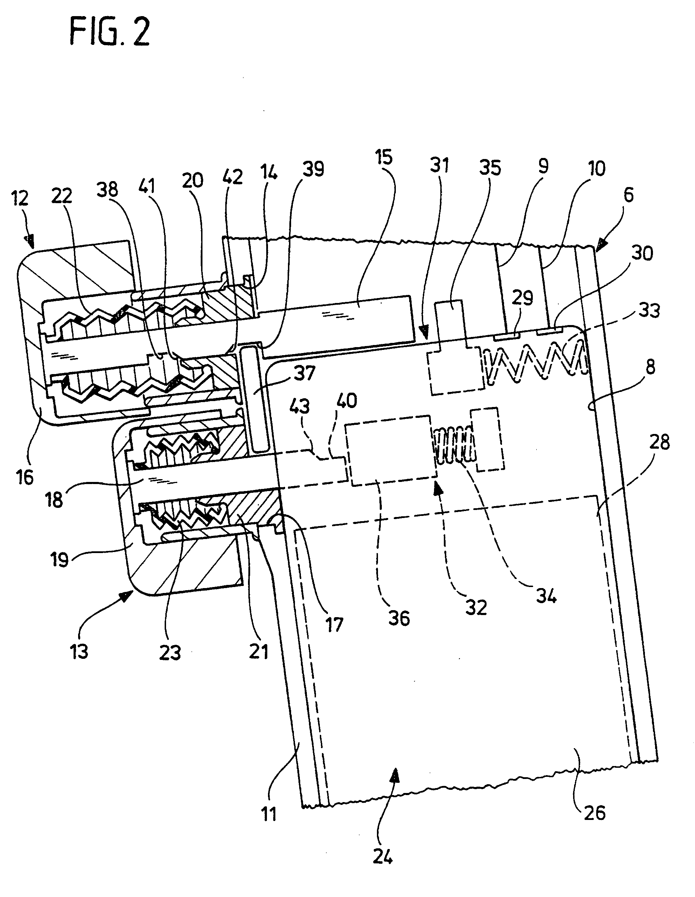

In Figur 2 ist die Betriebsstellung dargestellt, bei der der Elektromotor in einer Richtung umläuft. Der Richtungstaster 12 befindet sich in der ausgeschobenen Stellung, seine Ausnehmung 39 fluchtet mit dem Stift 37. Der Betriebstaster 13 kann in eine beliebige Position verfahren werden, in dieser Stellung kann also in eine Richtung mit beliebiger Ceschwindigkeit gebohrt werden.

Eine gleichzeitige Betätigung des Richtungstasters 12 ist unmöglich, da der Stift 37 nicht aus der Ausnehmung 39 ausgehoben werden kann.

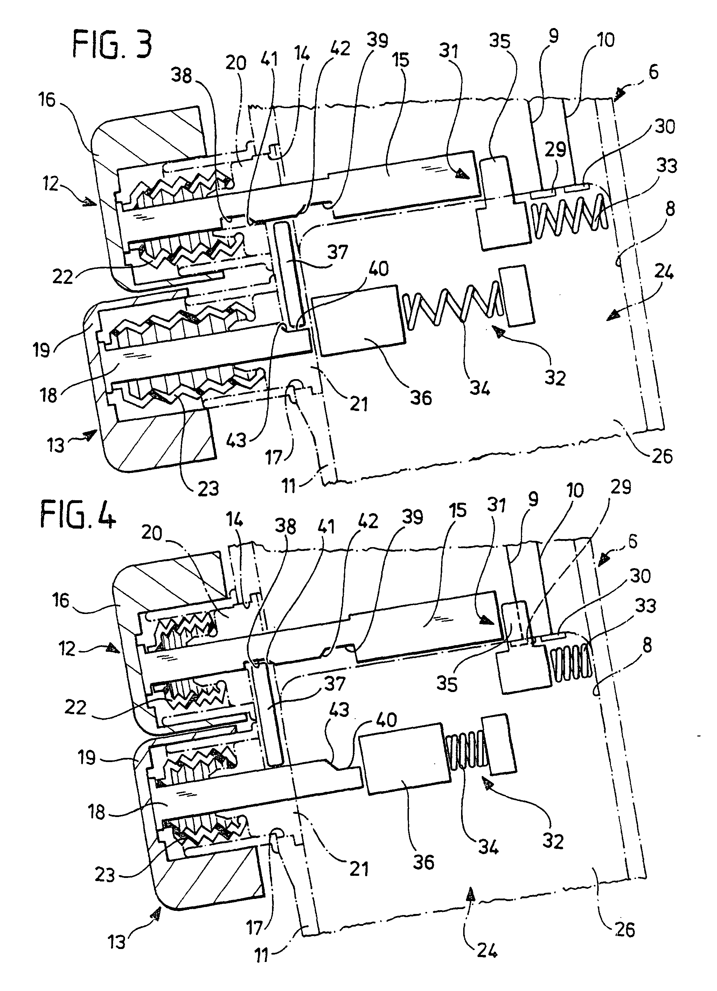

Dies ist erst wieder möglich, wenn sich der Betriebstaster 13 in seiner ausgeschobenen Ruhestellung befindet (Figur 3), dann kann der Stift 37 in die Ausnehmung 40 des unteren Stößels 18 eingreifen und gibt dadurch den oberen Stößel 15 frei, der Richtungstaster 12 kann betätigt werden. Eine gleichzeitige Betätigung des Betriebstasters 13 ist allerdings nicht möglich, bevor der Richtungstaster 12 vollständig eingedrückt ist, das heißt bevor der Stift 37 in die Ausnehmung 38 des Richtungstasters 12 eingreifen kann. Damit kann der Elektromotor erst in Betrieb gesetzt werden, wenn die Richtungsumschaltung abgeschlossen ist.

Figur 4 zeigt schließlich den Betrieb in dieser Betriebsweise mit umgeschalteter Richtung, der Stift taucht jetzt in die Ausnehmung 38 des oberen Stößels 15 ein und gibt dadurch den unteren Stößel 18 frei.

Durch diesen relativ einfachen Verriegelungsmechanismus wird sichergestellt, daß immer nur einer der beiden Taster 12 oder 13 betätigt werden kann.

## Patentansprüche

1. Bohrmaschine (1) für chirurgische Zwecke mit einem Gehäuse (2), in dem ein Elektromotor (3) und ein von diesem angetriebener Bohrmechanismus (4) angeordnet sind, und mit einem an das Gehäuse anschließenden Handgriff (6), an dem Steuergriffe (12) zur Geschwindigkeitssteuerung und zur Richtungsumschaltung des Elektromotors (3) angeordnet sind und der zur Aufnahme einer Batterie (27) und der Steuerelektrik (28) des Elektromotors dient, dadurch gekennzeichnet, daß in den Handgriff (6) ein als selbständig handhabbare Baueinheit ausgebildeter Energieblock (24) einsetzbar ist, der zusätzlich zu der Batterie (27) die Steuerelektrik (28), von den Steuergriffen (12, 13) betätigbare Schalter (31, 32) für die Richtungsumschaltung und die Geschwindigkeitssteuerung sowie elektrische Anschlüsse (29, 30) für den im Gehäuse (2) angeordneten Elektromotor (3) umfaßt.

2. Bohrmaschine nach Anspruch 1, dadurch gekennzeichnet, daß der Energieblock (24) eine untere Kammer (25) für die Batterie (27) und eine obere Kammer (26) für die Steuerelektrik (28) aufweist.

3. Bohrmaschine nach Anspruch 2, dadurch gekennzeichnet, daß die Schalter (31, 32) für die Richtungsumschaltung und für die Geschwindigkeitssteuerung in der oberen Kammer (26) angeordnet sind.

4. Bohrmaschine nach einem der voranstehenden Ansprüche, dadurch gekennzeichnet, daß der Energieblock (24) durch die geöffnete Unterseite des Handgriffs (6) in diesen einschiebbar ist.

5. Bohrmaschine nach einem der voranstehenden Ansprüche, dadurch gekennzeichnet, daß der Energieblock (24) die Schalter (31, 32) betätigende Betätigungsglieder (35 beziehungsweise 36) aufweist, an denen bei eingeschobenem Energieblock (24) die Steuergriffe (12, 13) anliegen und diese Betätigungsglieder (35 beziehungsweise 36) durch ihre eigene Bewegung bewegen.

6. Bohrmaschine nach Anspruch 5, dadurch gekennzeichnet, daß die Betätigungsglieder (35, 36) federbelastet in eine Ausgangsstellung bewegt werden.

7. Bohrmaschine nach Anspruch 5 oder 6, dadurch gekennzeichnet, daß ein Betätigungsglied (35) an der Oberseite des Energieblocks (24) aus diesem hervorsteht und quer zur Längsrichtung des Handgriffs (6) verschiebbar am Energieblock (24) gelagert ist.

8. Bohrmaschine nach einem der Ansprüche 5, 6 oder 7, dadurch gekennzeichnet, daß ein Betätigungsglied (36) im Inneren des Energieblocks (24) quer zur Längsrichtung des Handgriffs (6) verschiebbar gelagert ist und daß in der Vorderseite des Energieblocks (24) eine Durchbrechung vorgesehen ist, durch die hindurch ein Steuergriff (13) und das Betätigungsglied (36) aneinander anliegen.

9. Bohrmaschine nach einem der voranstehenden Ansprüche, dadurch gekennzeichnet, daß die Steuergriffe (12, 13) einen gegenseitigen Verriegelungsmechanismus (Stift 37; Ausnehmungen 38, 39, 40) aufweisen, der eine gleichzeitige Betätigung beider Steuergriffe (12, 13) verhindert.

10. Bohrmaschine nach einem der voranstehenden Ansprüche, dadurch gekennzeichnet, daß die Steuergriffe (12, 13) durch Öffnungen (14, 17) in der vorderen Wand (11) des Handgriffs (6) in dessen Innenraum (8) eintauchen und daß die Öffnungen (14, 17) durch die Steuergriffe (12, 13) umgreifende, flexible Membranen (22, 23) verschlossen sind.

11. Bohrmaschine nach Anspruch 10, dadurch gekennzeichnet, daß die Membranen (22, 23) die Form eines Faltenbalgs aufweisen.

## Claims

1. A drill (1) for surgical purposes, having a housing (2) in which an electric motor (3) and a drilling mechanism (4) driven by the latter are arranged, and having a handgrip (6) attached to the housing, control grips (12) for speed control and for direction reversal of the electric motor (3) being arranged on the handgrip (6) which serves to receive a battery (27) and the control electrics (28) of the electric motor, characterised in that an energy block (24) in the form of an independently manipulatable component is insertable into the handgrip (6) and, in addition to the battery (27), comprises the control electrics (28), switches (31, 32) - actuatable by the control grips (12, 13) - for the direction reversal and the speed control as well as electric connections (29, 30) for the electric motor (3) arranged in the housing (2).

2. A drill according to Claim 1, characterised in that the energy block (24) has a lower chamber (25) for the battery (27) and an upper chamber (26) for the control electrics (28).

3. A drill according to Claim 2, characterised in that the switches (31, 32) for direction reversal and for speed control are arranged in the upper chamber (26).

4. A drill according to any one of the preceding Claims, characterised in that the energy block (24) can be pushed into the handgrip (6) through the opened underside thereof.

5. A drill according to any one of the preceding Claims, characterised in that the energy block (24) has actuating members (35, 36) which actuate the switches (31 and 32 respectively), the control grips (12, 13) contacting the actuating members when the energy block (24) is inserted and moving these actuating members (35 and 36 respectively) through their own motion.

6. A drill according to Claim 5, characterised in that the actuating members (35, 36) are moved into an initial position by spring-loading.

7. A drill according to Claim 5 or 6, characterised in that an actuating member (35) projects from the energy block (24) at the upper side thereof and is mounted on the energy block (24) so as to be movable transversely to the longitudinal direction of the handgrip (6).

8. A drill according to any one of Claims 5, 6 or 7, characterised in that an actuating member (36) is mounted within the energy block (24) so as to be movable transversely to the longitudinal direction of the handgrip (6), and in that an opening is provided in the front of the energy block (24) and a control grip (13) and the actuating member (36) contact one another through this opening.

9. A drill according to any one of the preceding Claims, characterised in that the control grips (12, 13) have a reciprocal locking mechanism (pin 37; recesses 38, 39, 40) which prevents simultaneous actuation of both control grips (12, 13).

10. A drill according to any one of the preceding Claims, characterised in that the control grips (12, 13) project into the interior space (8) of the handgrip (6) through openings (14, 17) in the front wall (11) thereof, and in that the openings (14, 17) are closed by flexible membranes (22, 23) which embrace the control grips (12, 13).

11. A drill according to Claim 10, characterised in that the membranes (22, 23) have the form of bellows.

## Revendications

1. Perceuse (1) à usage chirurgical comportant un boîtier (2), dans lequel sont disposés un moteur électrique (3) et un mécanisme de perçage (4) entraîné par ce moteur, et une poignée (6) qui est raccordée au boîtier et sur laquelle sont disposés des organes de commande (12) pour commander la vitesse et inverser le sens de rotation du moteur électrique (3) et qui sert à loger une pile (27) et le système électrique de commande (28) du moteur électrique, caractérisée en ce que dans la poignée (6) peut être inséré un bloc d'alimentation en énergie (24), qui est agencé sous la forme d'une unité de construction pouvant être manipulée de façon autonome et qui comprend, en plus de la pile (27), le système électrique de commande (28), des commutateurs (31,32) pouvant être actionnés par les organes de commande (12,13) pour la commutation du sens de rotation et pour la commande de la vitesse, ainsi que des bornes électriques (29,30) pour le moteur électrique (3) disposé dans le boîtier (2).

2. Perceuse selon la revendication 1, caractérisée en ce que le bloc d'alimentation en énergie (24) comporte une chambre inférieure (25) pour la pile (27) et une chambre supérieure (26) pour le système électrique de commande (28).

3. Perceuse selon la revendication 2, caractérisée en ce que les commutateurs (31,32) pour l'inversion du sens de rotation et pour la commande de vitesse sont logés dans la chambre supérieure (26).

4. Perceuse selon l'une des revendications précédentes, caractérisée en ce que le bloc d'alimentation en énergie (24) peut être inséré dans la poignée (6), par le côté inférieur ouvert de cette dernière.

5. Perceuse selon l'une des revendications précédentes, caractérisée en ce que le bloc d'alimentation en énergie (24) comporte des organes d'actionnement (35 ou 36) qui actionnent les commutateurs (31,32) et sur lesquels les organes de commande (12,13) s'appliquent lorsque le bloc d'alimentation en énergie (24) est inséré et déplacent ces organes d'actionnement (35 ou 36) sous l'effet de leur propre déplacement.

6. Perceuse selon la revendication 5, caractérisée en ce que les organes d'actionnement (35,36) sont amenés dans une position de départ, en étant chargés par des ressorts.

7. Perceuse selon la revendication 5 ou 6, caractérisée en ce qu'un organe d'actionnement (35) est monté sur la face supérieure du bloc d'alimentation en énergie (24) de manière à faire saillie hors de ce dernier et est monté sur le bloc d'alimentation en énergie (24) de manière à être déplaçable transversalement par rapport à la direction longitudinale de la poignée (6).

8. Perceuse selon l'une des revendications 5, 6 ou 7, caractérisée en ce qu'un organe d'actionnement (36) est monté à l'intérieur du bloc d'alimentation en énergie (24) de manière à être déplaçable transversalement par rapport à la direction longitudinale de la poignée (6), et que dans la face avant du bloc d'alimentation en énergie (24) est prévu un passage à travers lequel un organe de commande (13) et l'organe d'actionnement (36) s'appliquent l'un contre l'autre.

9. Perceuse selon l'une des revendications précédentes, caractérisée en ce que les organes de commande (12,13) comportent un mécanisme de verrouillage réciproque (tige 37; évidements 38, 39, 40), qui empêche un actionnement simultané des deux organes de commande (12,13).

10. Perceuse selon l'une des revendications précédentes, caractérisée en ce que les organes de commande (12,13) pénètrent dans l'espace intérieur (8) de la poignée (6) par des ouvertures (14,17) aménagées dans la paroi avant (11) et que les ouvertures (14,17) sont fermées par des membranes flexibles (22,23), qui entourent les organes de commande (12,13).

11. Perceuse selon la revendication 10, caractérisée en ce que les membranes (22,23) possèdent la forme d'un soufflet.
